Europäisches Patentamt

(19) European Patent Office

Office européen des brevets

(11) Publication number : **0 565 236 A2**

(12) # EUROPEAN PATENT APPLICATION

(21) Application number : 93301711.3

(22) Date of filing : 08.03.93

(51) Int. Cl.⁵ : **A61K 39/02,** A61K 39/12, A61K 39/17, A61K 39/102, A61K 39/235, // A61K39/295

(30) Priority : 09.03.92 IL 101183

(43) Date of publication of application :
13.10.93 Bulletin 93/41

(84) Designated Contracting States :
AT BE CH DE DK ES FR GB GR IE IT LI LU MC NL PT SE

(71) Applicant : ABIC LIMITED
Industrial Zone Kiryat Nordau POB 8077
Natanya (IL)

(72) Inventor : Gutter, Bezalel
61 Shachal Street
Jerusalem (IL)

(74) Representative : Sanderson, Laurence Andrew et al
SANDERSON & CO. European Patent Attorneys 34, East Stockwell Street
Colchester Essex CO1 1ST (GB)

(54) Colour-coded inactivated vaccines for poultry.

(57) Inactivated poultry vaccines, e.g. Newcastle disease vaccine, Fowl Cholera disease vaccine and Turkey Haemorhagic Enteritis vaccine, are presented in a water-in-oil emulsion, wherein the aqueous, discontinuous phase is dyed with various dyes, e.g. Sunset Yellow, Brilliant Blue, green grass, Ponceau 4R and azorubine, or combinations of such dyes - the concentration of the dye(s) in the emulsion being at least 0.05 mg%.

EP 0 565 236 A2

Jouve, 18, rue Saint-Denis, 75001 PARIS

The present invention relates to inactivated vaccines (viral, bacterial or other) for poultry which have been colour-coded by staining with a distinctive dye.

Live vaccines for poultry, colour-coded by staining with a distinctive dye, are known from Israeli Patent Specification No.46189, which also describes the use and advantages of dye-stained virus vaccines.

The use of live vaccines is however not always satisfactory, and it is often advantageous instead to use inactivated vaccines, which have, inter alia, the following advantages:

a) there is no danger of back-mutation, which might occur in live attenuated vaccines; and

b) there is no danger of pathogenicity (vaccination reaction or side effects) whereas with live vaccines there is certain risk of pathogenicity.

Live vaccines are used in aqueous solution, and such aqueous solutions are dyed very easily, and require the use of only a small concentration of dye in the water, e.g. as low as 0.002 mg%.

However, inactivated vaccines are usually presented in the form of a water-in-oil (w/o) emulsion, and it has till now been believed that the dyes used to stain the live vaccines would not be effective in such w/o emulsions, and this view has been both reflected and confirmed by the fact that so far no coloured inactivated vaccines have become known.

It is therefore surprising that we have found that if the concentration of the dye in the aqueous phase is increased to at least 0.05 mg%, then such a w/o emulsion is quite effectively dyed and can be utilized in the same manner as the known dyed live vaccines, but it must be emphasised that the concentration of the dye in the final emulsion must in every case be at least 0.05 mg%, and may need to be still more, dependent on the dye employed.

It may here be noted that merely for convenience (in order to avoid having to use too many places of decimals) the dye-concentrations mentioned above and hereinafter are stated in "mg%", that is to say in terms of the number of milligrams of the dye(s) per 100 grams of the dyed w/o emulsion.

According to the present invention there is therefore now provided a colour-coded inactivated poultry vaccine in a water-in-oil (w/o) emulsion, in which the aqueous phase of said emulsion is dyed with a visually distinctive dye, the concentration of the dye in the emulsion being at least 0.05 mg%.

Quite unexpectedly, the use of an emulsion containing inactivated vaccine has been found to have certain further advantages, namely:

a) it confers a high degree of immunization, due presumably to an adjuvant effect of the oil part of the emulsion; and

b) it induces long and uniform immunity.

The w/o emulsion is suitably composed of:

an aqueous phase, which may be made up of water, saline solution, a buffer (such as phosphate buffered saline) or another type of innocuous solution;

an oil phase, which may be composed of a mineral oil (such as Drakeol, or another oil made up of various lengths of carbohydrate chains) or a vegetable oil (such as sesame, peanut and corn oils, etc.); and

emulsifiers for the aqueous phase, such as Tween 40, 60, 80, etc. (polyoxyethylene (POE) sorbitan monopalmitate, monostearate, monooleate, etc.,) and for the oil phase, such as Arlacel A, Arlacel 80, etc. (sorbitan monostearate, sorbitan monooleate, mannide monooleate, etc.).

Suitable dyes are, for example, those appearing in the following list:

| Name | Synonyms & Varieties |
| --- | --- |
| Sunset Yellow | C.I.Food Yellow 3; F.D. and V.C. Yellow No. 6 |
| Brilliant Blue | C.I.Food Blue 2; F.D. and C Blue No.1; Bleu Brilliant FCF |
| Green Grass | A mixture of Blue No. 1 FD & C, Brilliant Blue & Yellow No. 1 E 102 Tetrazine |
| Ponceau 4R | C.I.Food Red 7; Coccinel nonvelle, Cochineal Red A, E124 |
| Azorubine | C.I.Food Red 3; Carmuisine, E-122. |

as well as any combinations of the above dyes, but many other dyes may be employed, such as those approved for use as food dyes, or those used in the manufacture of pharmaceuticals.

The concentration of the dye in the emulsion varies according to the colour of the dye. Thus, the lowest desirable concentrations in the emulsion are, for example, respectively 0.05 mg% for the red emulsion, 0.5 mb% for the green emulsion and 0.25 mg% for the yellow emulsion.

In order that the invention may be well understood it will now be described in more detail though only by way of illustration, with reference to the following Examples:

EXAMPLE 1

Sunset Yellow dye solution was prepared by dissolving 100g of powder in 1 litre of bidistilled water. The solution was partitioned into bottles, stoppered with rubber stoppers and sealed with aluminium seals. It was then autoclaved at 121°C for 20 minutes and, after cooling, was tested for sterility.

This sterile solution was added at a concentration of 0.6% to ammnio-alantoic fluid (AAF) containing Komarov strain of Newcastle Disease virus - in the course of the otherwise-normal process for production of Inactivated Newcastle Disease Vaccine, ("Nectin").

The virus - dye solution together with a Tween 80 emulsifier was slowly incorporated into mineral oil containing Arlacel A, while mixing with a high-energy mixer (Silverson type).

Regular "Nectin" emulsion was compared to coloured "Nectin" emulsion by injecting 0.5 ml - intramuscularly (i.m.) into 4-week old chicks, and testing the level of antibodies in their serum 3 weeks later, using the haemaglutination inhibition method (H.I.).

The results thus obtained were as follows:

|  | HI Values (Log$_2$) | Average HI (Log$_2$) |
|---|---|---|
| Regular "Nectin": | 1x6, 2x7, 8x7 | $7.6 \pm 0.7$ |
| Yellow-coloured "Nectin": | 1x7, 9x8 | $7.9 \pm 0.3$ |
| Unvaccinated control: | 5x4, 2x5, 1x6 | $4.5 \pm 0.8$ |

Stability of the emulsion: No difference was observed between the coloured and the regular vaccine during storage for a period of 1.5 years at 4°C.

EXAMPLE 2

A green grass dye solution was prepared by dissolving 100g of dye powder in 1 litre of bidistilled water. The solution was partitioned into bottles, stoppered with rubber stoppers and sealed with aluminium seals. It was autoclaved at 121°C for 20 minutes and, after cooling, was tested for sterility.

This sterile solution was added at a concentration of 0.4% to **Pasteurella multocida** suspension for the preparation of Fowl Cholera Bacterin, ("Cholerin").

The bacteria-dye suspension together with a Tween 80 emulsifier was slowly incorporated into mineral oil containing Arlacel A, while mixing with a high-energy mixer (Silverson type).

Regular "Cholerin" emulsion was compared to coloured "Cholerin" emulsion by injecting turkeys with two doses of 0.5 ml, given at 5.5 weeks and 10 weeks of age, and then testing for their serum antibody level at 10 and 15 weeks of age, using the Agar-Gel Precipitin test.

The results thus obtained were as follows:

| | % Positive | | | | | | | |
|---|---|---|---|---|---|---|---|---|
| | First Bleeding | | | | Second Bleeding | | | |
| | PM1* | PM3 | PM4 | PA** | PM1 | PM3 | PM4 | PA |
| Regular "Cholerin": | 0 | 93 | 54 | 60 | 25 | 100 | 86 | 58 |
| Green-coloured "Cholerin": | 13 | 93 | 43 | 60 | 79 | 100 | 100 | 55 |
| Unvaccinated control: | 0 | 20 | 0 | 0 | 0 | 0 | 0 | 0 |

\*      PM 1, 3, 4 = **P.multocida** serotypes 1, 3 and 4.

\*\*     PA = **P.antipestifer.**

Stability of the emulsion: No difference was observed between the coloured and the regular "Cholerin" during storage for a period of 1.5 years at 4°C.

EXAMPLE 3

A Carmuisine (E122) red dye solution was prepared by dissolving 75g of dye powder in 1 litre of bidistilled water. The solution was partitioned into bottles, stoppered with rubber stoppers and sealed with aluminium seals. It was autoclaved at 121 °C for 20 minutes and, after cooling, was tested for sterility.

This sterile solution was added at a concentration of 0.8% to a spleen homogenate containing inactivated haemorhagic enteritis (H.E.) virus for the preparation of an inactivated HE vaccine, ("Damin").

The virus-dye solution together with a Tween 80 emulsifier was slowly incorporated into mineral oil containing Arlacel A, while mixing with a high-energy mixer (Silverson type).

Stability of the emulsion: No difference between the coloured and the regular "Damin" was observed during storage for a period of 1.5 years at 4°C.

Three week old turkeys were vaccinated with regular "Damin" in comparison with coloured "Damin" - both the coloured and uncoloured emulsions being prepared from identical antigen.

Vaccination was effected by injecting 0.5 ml intramuscularly. Three weeks post-vaccination, the !turkeys were challenged with pathogenic Haemorhagic Enteritis virus, and 4 days later the turkeys were sacrificed and their spleens were removed and investigated.

The spleens were tested for the presence or absence of HE virus. The absence of virus as well as a low spleen/body weight ratio indicates good immunization properties.

The results shown below clearly demonstrate that the coloured and the regular "Damin" both immunize turkeys equally well.

| | Presence of H.E. Virus in the Spleen | % Positive | Spleen/Body Weight Ratio |
|---|---|---|---|
| Regular "Damin" | 16/18 * | 89 | 0.13 |
| Coloured "Damin" | 16/16 | 100 | 0.12 |
| Unvaccinated control | 2/13 | 15 | 0.17 |

[Note: * 16 positive out of 18 tested, and similarly in other cases]

EXAMPLE 4

A purple dye solution was prepared by dissolving 30g of Carmuisine red dye and 10g of Brilliant Blue into 1 litre of bidistilled water.

A brown dye solution was achieved by mixing the above-mentioned purple dye solution with a Sunset Yellow solution as described in Example 1 and with a Carmuisine red solution as described in Example 3, the mixing ratio being - purple : red : yellow = 1:1:3.

This brown solution was added - at a concentration of 0.8% - to a homogenate of Bursae of Fabricius tissue infected with infectious bursal disease virus (IBD), in the course of the normal preparation of a combined vaccine against Gumboro (IBD) and Newcastle disease inactivated vaccine ("Bursatin").

The brown IBD-virus solution was mixed with AAF containing Newcastle disease virus, and an emulsifying agent (Tween 80) was added. The mixture was slowly poured into a mineral oil phase containing Arlacel A while mixing with a high-energy homogenizer.

Stability of the emulsion: No difference was observed between the coloured and the regular "Bursatin" during storage for a period of 1.5 years at 4°C.

Regular "Bursatin" was compared to dyed "Bursatin" by injecting 0.4 ml i.m. into 1-week old chicks, and testing their serum antibody level 3.5 weeks later.

In addition, the chicks were infected by inoculating them with virulent IBDV field strain. Resistance to infection was proved by lack of IBD virus in the Bursae of Fabricius 3 days post-challenge.

The results thus obtained were as follows:

| Presence of IBDV in Bursae post-challenge | Serum NDV HI log$_2$ | Serum IBD AGP ** | Antibody Levels Elisa *** |
|---|---|---|---|
| Reg."Bursatin" | 14/15 * | 6.7 $\pm$ 0.6 | 11/15 | 12/15 |
| Dyed "Bursatin" | 14/15 | 6.5 $\pm$ 0.7 | 11/15 | 11/15 |
| Unvaccinated control | 0/15 | 3.2 $\pm$ 0.5 | 0/14 | 0/14 |

[Notes:

\*      14 positive for presence of virus out of 15 tested, and similarly in other cases.

\*\*      AGP = agar gel precipitin test.

\*\*\*      Elisa = enzyme-linked immuno-sorbent assay.].

**Claims**

1. A colour-coded inactivated poultry vaccine in a water-in-oil (w/o) emulsion, in which the aqueous phase of said emulsion is dyed with a visually distinctive dye, the concentration of the dye in the emulsion being at least 0.05 mg%.

2. A colour-coded inactivated poultry vaccine as claimed in claim 1, in which the dye is or includes one or a mixture of more than one of the following viz: Sunset viz: Yellow, Brilliant Blue, green grass, Ponceau 4R and/or azorubine.

3. Inactivated Newcastle disease vaccine as claimed in claim 1 or claim 2, which is coloured with Sunset Yellow dye in a concentration of at least 0.25 mg%.

4. Inactivated Fowl Cholera vaccine as claimed in claim 1 or claim 2, which is coloured with green grass dye in a concentration of at least 0.5 mg%.

5. Inactivated turkey Haemorhagic Enteritis vaccine as claimed in claim 1 or claim 2, which is coloured with Carmuisine dye in a concentration of at least 0.05 mg%.